Europäisches Patentamt

European Patent Office

Office européen des brevets

(11) Numéro de publication: **0 252 059**
**A2**

# (12) DEMANDE DE BREVET EUROPEEN

(21) Numéro de dépôt: 87870090.5

(51) Int. Cl.4: **A 61 K 39/12**

(22) Date de dépôt: 29.06.87

(30) Priorité: 30.06.86 US 880375

(43) Date de publication de la demande:
07.01.88 Bulletin 88/01

(84) Etats contractants désignés:
AT BE CH DE ES FR GB GR IT LI LU NL SE

(71) Demandeur: Smith Kline - RIT Société anonyme dite:
rue du Tilleul, 13
B-1320 Genval (Rixensart) (BE)

(72) Inventeur: D'Hondt, Erik
Schoolstraat 25
B-3052 Ottenburg (BE)

(74) Mandataire: Tasset, Gérard
SMITHKLINE - RIT rue de l'Institut, 89
B-1330 Rixensart (BE)

(54) Stabilisants améliorés pour vaccins atténués, procédé pour leur préparation et vaccins les contenant.

(57) Des stabilisants pour vaccins atténués sont préparés à base de lactose, de sorbitol, de dextranne, d'hydrolysat de caséine, d'acide L-glutamique ou d'un de ses sels alcalins, d'acide éthylène diamine tétraacétique ou d'un de ses sels alcalins et d'une solution tampon pharmaceutiquement acceptable à un pH compris entre 6,7 et 7,2 dans de l'eau distillée apyrogène.

EP 0 252 059 A2

## Description

Stabilisants améliorés pour vaccins atténués, procédé pour leur préparation et vaccins les contenant

Domaine de l'invention

La présente invention concerne des stabilisants améliorés pour vaccins atténués, par exemple le vaccin contre le virus herpes zoster de la varicelle, un procédé pour leur préparation et les vaccins qui les contiennent.

Les spécifications exigées pour les vaccins demandent qu'ils soient et demeurent sans danger et efficaces pendant une période acceptable du point de vue commercial et dans des conditions de stockage pratiquement valables. Le problème est important car il arrive souvent que l'ingrédient actif soit très sensible à une dégradation possible en cours de production ou de transport. C'est pourquoi il est habituellement recommandé de stocker les vaccins à une température comprise entre 2 et 4° C ou même à - 20° C dans le cas d'un vaccin contre la varicelle. Cette fragilité est particulièrement marquée pour les vaccins atténués. Par conséquent, les vaccins du commerce contiennent généralement un composant qui est soit un produit unique soit, plus fréquemment, une composition assez complexe, chacun de ces composants étant ici désigné comme 'stabilisant' dont la nature varie suivant la nature de l'ingrédient actif.

Le virus herpes zoster de la varicelle est un virus thermolabile particulièrement sensible aux conditions dégradatives mentionnées ci-avant. Dans les cultures de cellules, l'infection par le virus de la varicelle est, de manière prédominante, liée aux cellules et la récolte de particules infectieuses après le stade de lyophilisation est insatisfaisante et on constate une perte d'infectivité significative après un bref stockage du virus lyophilisé à température ordinaire.

Arrière-plan technologique

De nombreux stabilisants ont été proposés pour différents vaccins.

Plus particulièrement, la littérature des brevets fournit les références suivantes :

- le brevet européen 0 028 563 qui décrit un vaccin viral sous forme liquide comprenant un virus atténué (par exemple le virus de la varicelle) et un stabilisant constitué de gélatine partiellement hydrolysée, d'un mono- ou di-saccharide, d'un milieu de culture cellulaire in vitro, d'acide L'glutamique, de L-arginine et d'une quantité d'un tampon physiologiquement acceptable efficace pour le maintien d'une valeur pH comprise entre 6,0 et 6,5.

- le brevet français 2 076 787 qui décrit un stabilisant de lyophilisation du virus de la rubéole contenant des anions phosphoriques, des cations potassium, du lactose, de l'albumine et de l'acide glutamique.

- le brevet 2 406 167 de la République Fédérale d'Allemagne qui décrit des suspensions stables de virus (notamment du virus de la varicelle) préparées en traitant des cellules infectées avec une solution tampon contenant de la polyvinylpyrrolidone, avant la rupture des cellules infectées par le virus.

- le brevet 3 783 098 des Etats-Unis d'Amerique qui décrit une composition stabilisante pour virus herpes Groupe B, herpes dinde ou de la maladie de Marek constituée de protéines, plus spécialement albumine ou caséine et/ou un dérivé d'amidon.

- le brevet 4 000 256 des Etats-Unis d'Amérique qui donne l'exemple d'un procédé pour stabiliser un vaccin non cellulaire contre la varicelle dans lequel les cellules infectées sont soumises à un traitement ultrasonique en présence d'un stabilisant contenant du saccharose, de l'albumine, de la glutamine et du phosphate.

- le brevet européen 0 008 255 qui décrit une composition stabilisante pour différents virus (entre autres le virus herpes zoster de la varicelle) constituée de saccharose, d'un tampon phosphate, de L-glutamate monosodique et d'albumine bovine.

- le brevet 4 147 772 des Etats-Unis d'Amérique qui décrit un stabilisant contenant un hydrolysat de gélatine, du sorbitol, un milieu de culture et un tampon à pH 6-6,5.

- le brevet européen 0 030 199 qui concerne une méthode de lyophilisation d'une composition virale aqueuse en présence d'un tampon phosphate et, soit de saccharose, de glutamate monosodique et d'albumine bovine, soit d'hydrolysat de gélatine et de sorbitol.

- le brevet européen 0 065 905 qui décrit un agent de stabilisation pour vaccins contenant des virus atténués et comprenant un tampon phosphate contenant des ions calcium et magnésium, du lactose, du sorbitol et au moins un acide aminé.

- le brevet belge 861 265 qui concerne la stabilisation d'un vaccin atténué contre la polyomyélite à l'aide de tris-hydroxyméthyl-amino-méthane, de L-cystine et, si on le désire, d'hydrolysat de gélatine.

- le brevet belge 664 429 qui décrit la stabilisation de vaccins BCG par un mélange de saccharose, de dextranne et de glutamate monosodique.

Résumé de l'invention

La présente invention fournit des nouveaux stabilisants pour vaccins atténués, un procédé pour leur préparation et des vaccins soit monovalents soit multivalents contenant lesdits stabilisants.

Lorsqu'ils sont comparés aux stabilisants précédemment connus, les nouveaux stabilisants de l'invention montrent des propriétés stabilisantes améliorées pendant la lyophilisation et le stockage du matériel viral, rendant, par exemple, possible le stockage d'un vaccin varicelle à + 4° C au lieu de - 20° C.

## Description détaillée de l'invention

Comme il est indiqué plus haut, le virus zoster de la varicelle (également cité ici sous l'appellation virus de la varicelle) est un virus thermolabile et, après lyophilisation, sa récolte de particules infectées est faible. Ces caractéristiques le rendent particulièrement intéressant comme virus test dans des études d'efficacité de stabilisation.

Dans la présente description, le virus de la varicelle a été utilisé dans des essais comparatifs effectués en relation avec la présente invention, laquelle n'est en aucune manière limitée au seul virus de la varicelle.

L'invention fournit donc des nouveaux stabilisants pour vaccins viraux, plus particulièrement vaccins viraux atténués et, suivant un autre aspect, l'invention concerne également les vaccins viraux - plus particulièrement viraux atténués - contenant de tels stabilisants.

Les stabilisants de cette invention consistent essentiellement en une solution aqueuse de lactose (30-50 g); d'un monosaccharide, par exemple du sorbitol (15-35 g); de dextranne (de préférence d'un poids moléculaire de 10.000) (15-40 g); d'hydrolysat de caséine (10-30 g); d'acide L-glutamique et de glutamate monosodique (1-3 g); d'acide éthylène diamine tétraacétique ou de son sel sodique (0,2-1 g) et d'une quantité d'un tampon physiologiquement acceptable efficace pour le maintien d'une valeur pH comprise entre 6,7 et 7,2, par exemple un tampon phosphate, un tampon acétate ou un tampon citrate, de préférence un tampon phosphate, par exemple constitué de phosphate disodique (0,5-2 g) et de phosphate monopotassique (0,3-1 g), toutes les quantités étant indiquées pour un litre d'eau distillée apyrogène.

Comme indiqué plus haut, ledit stabilisant peut être utilisé pour des vaccins viraux atténués, par exemple contre la rougeole, les oreillons, la rubéole et la varicelle, soit sous forme séparée soit en combinaison.

Suivant un autre aspect encore de la présente invention, le stabilisant est préparé en dissolvant les différents ingrédients dans de l'eau distillée apyrogène et la solution est ajoutée à la préparation virale de préférence à la fin de la croissance du virus, avant toute concentration, adsorption, sonication ou lyophilisation de la préparation virale. Par exemple, dans le cas de vaccin contre la varicelle, l'infection virale en culture de cellules est, de manière prédominante, liée aux cellules et par congélation et décongélation répétées ou par traitement aux ultrasons, le virus passe alors des cellules infectées dans le stabilisant testé.

L'invention est illustrée par les exemples suivants qui n'en limitent pas la portée.

## Exemple 1

### a) Procédé général de culture du virus de la varicelle

Comme il est bien connu en pratique, le virus de la varicelle peut, en cultures cellulaires, être isolé de vésicules d'un cas clinique de varicelle, en utilisant un type de cellules approprié tel que des cellules primaires humaines, cellules diploïdes humaines et cellules de primates et le virus peut être atténué par passages en série dans différentes cultures cellulaires.

La multiplication du virus atténué est alors effectuée dans un substrat de culture cellulaire (par exemple dans des cellules de la lignée cellulaire MRC5). Après incubation à une température comprise entre 34° C et 37° C et lorsqu'un effet cytopathogène prononcé indique un état avancé d'infection, on arrête la culture.

### (b) Préparation du stabilisant

On prépare un stabilisant en dissolvant les ingrédients suivants dans de l'eau distillée apyrogène, toutes les quantités étant calculées pour un litre d'eau distillée apyrogène.

Lactose 90 g
Sorbitol 50 g
Dextranne 10.000 56 g
Hydrolysat de caséine 42 g
Glutamate monosodique 4 g
$Na_2HPO_4$ 2 g
$KH_2PO_4$ 1 g

### (c) Préparation de vaccin stabilisé

Le milieu de culture cellulaire obtenu à la fin de l'Exemple 1(a) est écarté et les cellules infectées recueillies sont lavées trois fois pour éliminer tout composant du milieu de culture. Les lavages sont effectués avec la préparation stabilisante de l'Exemple 1 (b).

Les cellules sont détachées de la paroi du récipient à l'aide d'une solution aqueuse de sel sodique d'EDTA (0,4 g/l) et mises en suspension dans le stabilisant de l'Exemple 1(b), la concentration finale étant 50 %.

Le virus est libéré des cellules infectées par sonication (pendant 90 secondes à 50 % de la puissance d'un disrupteur de cellules BRANSON B30) et la suspension virale obtenue est stockée à - 70° C.

Des quantités aliquotes de la suspension virale stockée à - 70° C sont dégelées et lyophilisées dans des récipients en verre qui sont ensuite bouchés hermétiquement.

### (d) Test de stabilité

Différentes quantités aliquotes de lots de vaccins préparés comme indiqué ci-avant ont été stockées soit pendant 7 jours à 37° C soit pendant une année à + 4° C et on a déterminé la perte d'infectivité.

Les résultats sont résumés dans le Tableau 1 suivant :

## Tableau I

| Lot No. | Perte du titre d'infectivité (en $\log_{10}$) | |
| --- | --- | --- |
| | après 7 jours à 37° C | après une année à 4° C |
| 1188-A | 0,62 | 0,1 |
| 1194 | 0,59 | 0,1 |
| 1198-A | | 0,21 |
| 1198-B | | 0,34 |
| 1345 | 0,70 | |
| 1358 | 0,33 | |

### Exemple 2

Une culture cellulaire de virus de la varicelle tel qu'obtenu à la fin de l'Exemple 1(a) est séparée du milieu de culture cellulaire et les cellules infectées récoltées sont traitées comme indiqué dans l'Exemple 1(c) mais en utilisant, à 100 %, un stabilisant consistant en une solution aqueuse de saccharose (74,5 g); de glutamate monopotassique (0,8 g); de phosphate disodique (1 g); de phosphate monopotassique (0,52 g); d'EDTA (0,2 g) et de polyvinylpyrrolidone K15 (10 g) par litre d'eau distillée apyrogène, en lieu et place du stabilisant indiqué dans l'Exemple 1(b).

Le vaccin obtenu finalement a été testé au point du vue de sa stabilité, dans les mêmes conditions que celles qui sont indiquées dans l'Exemple 1(d).

Les résultats sont résumés dans le Tableau II suivant :

## Tableau II

| Lot No. | Perte du titre d'infectivité (en $\log_{10}$) après 7 jours à 37° C |
| --- | --- |
| L-688 | 1,51 |
| L-716 | 0,97 |
| L-699 | 1,07 |

### Exemple 3

Une culture cellulaire de virus de la varicelle tel qu'obtenu à la fin de l'Exemple 1(a) est séparée du milieu de culture cellulaire et les cellules infectées récoltées sont traitées comme indiqué dans l'Exemple 1(c) mais en utilisant, à 100 %, un stabilisant consistant en une solution aqueuse de lactose (40 g); de sorbitol (35 g); de dextranne (30 g); d'hydrolysat de caséine (21 g) et d'albumine humaine (2 g) par litre d'eau distillée apyrogène, en lieu de place du stabilisant indiqué dans l'Exemple 1(b).

Le vaccin obtenu finalement a alors été testé au point de vue de sa stabilité dans les mêmes conditions que celles qui sont indiquées dans l'Exemple 1(d).

Les résultats sont résumés dans le Tableau III suivant :

## Tableau III

| Lot No. | Perte du titre d'infectivité (en $\log_{10}$) après 7 jours à 37° C |
|---------|---------|
| U-1226 | 0,94 |
| U-1243 | 1,08 |
| U-1246 | 1,00 |
| U-1305 | 0,86 |

Exemple 4

Une récolte de virus des oreillons atténué (souche URABE AM9); une récolte de virus de la rubéole atténué (souche RA 27/3) et une récolte de virus de la rougeole atténué (souche SCHWARTZ) ont été obtenus séparément, respectivement au départ de cultures sur fibroblastes d'embryons de poulets, de cellules MRC5 et de fibroblastes d'embryons de poulets, en utilisant des techniques bien connues dans la pratique.

Chaque récolte a été additionnée d'un stabilisant constitué de lactose (90 g), de sorbitol (70 g) et de dextranne (70 g) par litre d'eau distillée apyrogène, le rapport stabilisant/récolte étant dans chaque cas 50/50 (v/v) et les 3 compositions ont été stockées à - 45° C.

Après avoir dégelé les 3 récoltes de virus, celles-ci ont été mélangées à une récolte de virus de la varicelle préparé comme indiqué dans l'Exemple 1(c) et à une préparation concentrée de stabilisant de l'Exemple 1(b) de manière à obtenir des quantités et proportions respectives de chaque composant qui soient acceptables pour un usage vaccinal, c'est-à-dire 10 % de virus de la rougeole; 20 % de virus des oreillons; 20 % de virus de la rubéole et 50 % de virus de la varicelle et pour obtenir comme concentrations finales des composants du stabilisant : lactose (45 g), sorbitol (25 g), dextranne (28 g), hydrolysat de caséine (21 g), glutamate monosodique (2 g), sel sodique de l'acide éthylène diamine tétraacétique (0,2 g), phosphate disodique (1 g) et phosphate monopotassique (0,5 g) pour un litre de vaccin tétravalent.

Exemple 5

Deux lots de vaccin tétravalent préparés par la méthode de l'Exemple 4 ont été lyophilisés et ensuite stockés pendant 7 jours à 37° C, après quoi la perte du titre d'infectivité a été déterminée pour chaque virus.

Les résultats sont résumés dans le Tableau IV qui montre que chaque virus a subi une perte d'infectivité particulièrement faible.

## Tableau IV

| Lot No. | Perte du titre d'infectivité (en $\log_{10}$) | | | |
|---------|-----------|----------|----------|---------|
| | Varicelle | Rougeole | Oreillons | Rubéole |
| 1345 | 0,55 | 0,48 | 0,50 | 0,04 |
| 1358 | 0,59 | 0,60 | ND | 0,17 |
| 01B42 | 0,65 | 0,44 | 0,49 | 0,12 |

ND : non déterminé

**Revendications**

1. Stabilisant pour vaccins contenant des virus atténués caractérisé en ce qu'il comprend du lactose, du sorbitol, du dextranne, de l'hydrolysat de caséine, de l'acide L-glutamique ou un de ses sels alcalins, de l'acide éthylène diamine tétraacétique ou un de ses sels alcalins et une solution tampon pharmaceutiquement acceptable à un pH compris entre 6,7 et 7,2 dans de l'eau distillée apyrogène.

2. Stabilisant pour vaccins suivant la revendication 1 caractérisé en ce qu'il contient du lactose, du sorbitol, du dextranne, de l'hydrolysat de caséine, du L-glutamate de sodium, du sel sodique de l'acide éthylène diamine tétraacétique et un tampon phosphate monopotassique à des concentrations respectives de 30-50 g; 15-35 g; 15-40 g; 10-30 g; 1-3 g; 0,2-1 g; 0,5-2 g et 0,3-1 g par litre d'eau distillée apyrogène.

3. Vaccin stabilisé contenant des virus atténués constitué essentiellement d'une suspension de virus atténués dans un stabilisant suivant la revendication 1 ou 2, sous forme lyophilisée.

4. Vaccin stabilisé suivant la revendication 3, caractérisé en ce qu'il contient du virus herpes zoster de la varicelle atténué.

5. Vaccin stabilisé suivant la revendication 3, caractérisé en ce qu'il contient du virus de la rougeole atténué.

6. Vaccin stabilisé suivant la revendication 3, caractérisé en ce qu'il contient du virus des oreillons atténué.

7. Vaccin stabilisé suivant la revendication 3, caractérisé en ce qu'il contient du virus de la rubéole atténué.

8. Procédé de préparation d'un vaccin stabilisé suivant l'une ou l'autre des revendications 3 à 7, caractérisé en ce qu'on met en suspension une quantité appropriée dudit virus dans un stabilisant qui comprend du lactose, du sorbitol, du dextranne, de l'hydrolysat de caséine, de l'acide L-glutamique ou un de ses sels alcalins, de l'acide éthylène diamine tétraacétique ou un de ses sels alcalins et une solution tampon pharmaceutiquement acceptable à un pH compris entre 6,7 et 7,2 dans de l'eau distillée apyrogène.

9. Procédé de préparation d'un vaccin stabilisé suivant la revendication 8 dans lequel le stabilisant comprend du lactose, du sorbitol, du dextranne, de l'hydrolysat de caséine, du L-glutamate de sodium, du sel sodique de l'acide éthylène diamine tétraacétique et un tampon phosphate fait de phosphate disodique et de phosphate monopotassique à des concentrations respectives de 30-50 g; 15-35 g; 15-40 g; 10-30 g; 1-3 g; 0,2-1 g; 0,5-2 g et 0,3-1 g par litre d'eau distillée apyrogène.

Revendications pour les Etats suivants: AT, ES, GR 1. Procédé de préparation d'un vaccin viral atténué stabilisé, caractérisé en ce qu'on met en suspension une quantité appropriée dudit virus dans un stabilisant qui comprend du lactose, du sorbitol, du dextranne, de l'hydrolysat de caséine, de l'acide L-glutamique ou un de ses sels alcalins, de l'acide éthylène diamine tétraacétique ou un de ses sels alcalins et une solution tampon pharmaceutiquement acceptable à un pH compris entre 6,7 et 7,2 dans de l'eau distillée apyrogène.

2. Procédé de préparation d'un vaccin viral atténué suivant la revendication 1 dans lequel on met en suspension une quantité appropriée dudit virus dans un stabilisant qui comprend du lactose, du sorbitol, du dextranne, de l'hydrolysat de caséine, du L-glutamate de sodium, du sel sodique de l'acide éthylène diamine tétraacétique et un tampon phosphate fait de phosphate disodique et de phosphate monopotassique à des concentrations respectives de 30-50 g; 15-35 g; 15-40 g; 10-30 g; 1-3 g; 0,2-1 g; 0,5-2 g et 0,3-1 g par litre d'eau distillée apyrogène.